(19) Europäisches Patentamt
European Patent Office
Office européen des brevets

(11) Veröffentlichungsnummer: **0 085 333**
**B1**

(12) # EUROPÄISCHE PATENTSCHRIFT

(45) Veröffentlichungstag der Patentschrift: **26.02.86**

(51) Int. Cl.⁴: **C 07 D 249/08**, C 07 D 233/60, A 01 N 43/64, A 01 N 43/50

(21) Anmeldenummer: **83100337.1**

(22) Anmeldetag: **17.01.83**

(54) **Ether-Derivate von substituierten 1-Hydroxyalkyl-azolen, Verfahren zu ihrer Herstellung sowie ihre Verwendung als Fungizide und Pflanzenwachstumsregulatoren.**

(30) Priorität: **27.01.82 DE 3202604**

(43) Veröffentlichungstag der Anmeldung: **10.08.83 Patentblatt 83/32**

(45) Bekanntmachung des Hinweises auf die Patenterteilung: **26.02.86 Patentblatt 86/9**

(84) Benannte Vertragsstaaten: **AT BE CH DE FR GB IT LI NL SE**

(56) Entgegenhaltungen:
**EP - A - 0 052 424**
**EP - A - 0 061 835**
**DE - A - 2 737 489**
**US - A - 4 123 542**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Holmwood, Graham, Dr., Katernberger Strasse 184, D-5600 Wuppertal 1 (DE)**
Erfinder: **Regel, Erik, Ing.-grad., Untere Bergerheide 26, D-5600 Wuppertal 1 (DE)**
Erfinder: **Jäger, Gerhard, Dr., Gellertstrasse 18, D-5090 Leverkusen 1 (DE)**
Erfinder: **Büchel, Karl Heinz, Prof. Dr., Dabringhausener Strasse 42, D-5093 Burscheid (DE)**
Erfinder: **Frohberger, Paul-Ernst, Dr., Willi-Baumeister-Strasse 5, D-5090 Leverkusen 1 (DE)**
Erfinder: **Brandes, Wilhelm, Dr., Eichendorffstrasse 3, D-5653 Leichlingen 1 (DE)**
Erfinder: **Lürssen, Klaus, Dr., August-Kierspel-Strasse 89, D-5060 Bergisch-Gladbach 2 (DE)**

**EP 0 085 333 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue Ether-Derivate von substituierten 1-Hydroxyalkyl-azolen, ein Verfahren zu ihrer Herstellung sowie ihre Verwendung als Fungizide und Pflanzenwachstumsregulatoren.

Es ist bereits bekannt geworden, daß bestimmte Biphenylylhydroxyalkyl-azol-Derivate, wie beispielsweise 2-(4-Bi-phenylyl)-1-(2,4-dichlorphenyl)- bzw. -phenyl-3-(1,2,4-triazol-1-yl)-2-propanol und 4-Biphenylyl-2-chlor- bzw. fluorphenyl-(1,2,4-triazol-1-yl—methyl)-carbinol, gute fungizide und in bestimmten Aufwandmengen auch pflanzenwachstumsregulierende Eigenschaften aufweisen (vergleiche DE-OS 27 37 489 und DE-OS 29 20 374). Die Wirksamkeit dieser Verbindungen ist jedoch, insbesondere bei niedrigen Aufwandmengen und -konzentrationen, nicht immer ganz befriedigend. Weiterhin sind Ether-Derivate von bestimmten Hydroxyalkyl-imidazolen aus der Patentliteratur bekannt (vgl. US-PS 4 123 542 und 4 277 475), die eine Wirkung gegen humanpathogene und auch gegen pflanzenpathogene Pilze aufweisen und als Arzneimittel und zudem auch als Contrazeptiva verwendet werden können (US-PS 4 277 475). Ferner ist bereits bekannt geworden, daß bestimmte Phenyl-triazolyl-ethanol-Derivate, wie beispielsweise 1-(4-Chlorphenyl)-2-(1,2,4-triazol-1-yl)-1-ethanol, fungizide Eigenschaften besitzen (vgl. DE-OS 24 31 407). Die Wirkung dieser Verbindungen ist ebenfalls, insbesondere bei niedrigen Aufwandmengen und -konzentrationen, nicht immer befriedigend. Dasselbe gilt auch für Ethyl- und Propylazolyl-Derivate, die in zwei älteren, aber nicht vorveröffentlichten Patentanmeldungen beschrieben sind (vgl. EP-A 0 061 835 und EP-A 0 052 424).

Es wurden neue Ether-Derivate von substituierten 1-Hy-droxyalkyl-azolen der allgemeinen Formel

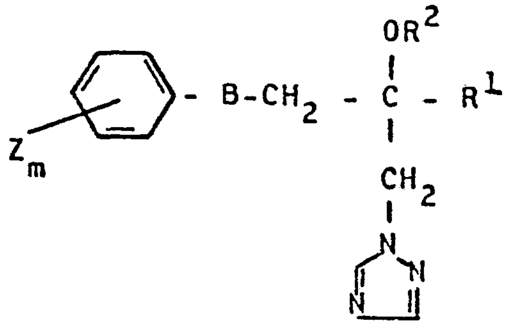

(I)

in welcher

$R^1$ für tert.-Butyl, Isopropyl oder Methyl steht weiterhin für die Gruppierungen

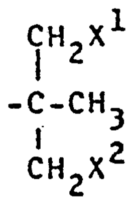

und

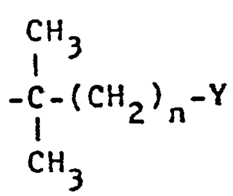

steht, sowie für gegebenenfalls durch Fluor, Chlor, Methyl oder Trifluormethyl ein- bis dreifach substituiertes Phenyl steht; wobei

$X^1$ für Wasserstoff, Fluor, Chlor oder Brom steht;

$X^2$ für Fluor, Chlor oder Brom steht;

Y für Methyl, Ethyl, Propyl, Methoxy, Ethoxy, Vinyl, sowie für gegebenenfalls ein- bis dreifach durch Chlor an den Phenyl-Resten substituiertes Phenyl oder Phenoxy steht;

n für die Zahlen 0 oder 1 steht;

Z für Fluor, Chlor, Brom, Methyl, tert.-Butyl, Methoxy, Trifluormethyl, Trifluormethoxy, sowie für gegebenenfalls durch Chlor ein- bis dreifach substituiertes Phenyl steht;

$R^2$ für Methyl, Ethyl, n-Propyl, Isopropyl, n-Butyl Isobutyl, Allyl und Propargyl steht;

B für Schwefel oder die $CH_2$-Gruppe steht und weiterhin noch für Sauerstoff steht, sofern Z nicht Fluor bedeutet; und

m für die Zahlen 0, 1, 2 oder 3 steht;

sowie deren Säureadditions-Salze und Metallsalz-Komplexe gefunden.

Die Verbindungen der Formel (I) besitzen ein asymmetrisches Kohlenstoffatom und können deshalb in den beiden optischen Isomerenformen anfallen.

Weiterhin wurde gefunden, daß man die Ether-Derivate von substituierten 1-Hydroxyalkyl-azolen der Formel (I) erhält, wenn man Alkoholate von 1-Hydroxyalkyl-azolen der Formel

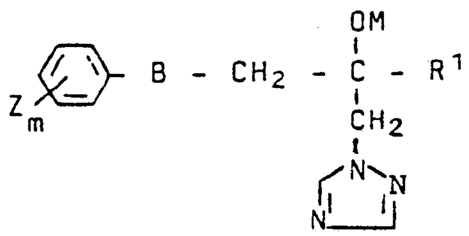

(II)

in welcher

B,$R^1$,Z und m die oben angegebene Bedeutung haben und

M für ein Alkalimetall, eine quarternäre Ammonium- oder Phosphoniumgruppe steht,

mit einem Halogenid der Formel

$R^2$ - Hal (III)

in welcher

$R^2$ die oben angegebene Bedeutung hat und

Hal für Halogen steht,

in Gegenwart eines Verdünnungsmittels umsetzt, und gegebenenfalls anschließend an die so erhaltenen Verbindungen der Formel (I) eine Säure oder ein Metallsalz addiert.

Außerdem wurde gefunden, daß die neuen Ether-Derivate von substituierten 1-Hydroxyalkyl-azolen der Formel (I) starke fungizide und starke pflanzenwachstumsregulierende Eigenschaften besitzen.

Ueberraschenderweise zeigen die erfindungsgemäßen Ether-Derivate der Formel (I) bessere fungizide und bessere pflanzenwachstumsregulierende Wirkungen ala die oben genannten, aus dem Stand der Technik bekannten, Triazolyl-Derivate. Die erfindungsgemäßen Wirkstoffe stellen somit eine Bereicherung der Technik dar.

Die erfindungsgemäßen Ether-Derivate von substituierten 1-Hydroxyalkyl-azolen sind durch die Formel (I) allgemein definiert.

Erfindungsgemäße Verbindungen sind auch Additionsprodukte aus Säuren und denjenigen Ether-Derivaten von substituierten 1-Hydroxyalkyl-azolen der Formel (I), in denen die Substituenten B, $R^1$, $R^2$ und $Z_m$ die Bedeutungen haben, die bereits für diese Substituenten genannt wurden.

Zu den Säuren,die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalin-disulfonsäure.

Außerdem sind erfindungsgemäße Verbindungen Additionsprodukte aus Salzen von Metallen der II. bis IV. Haupt- und der I. und II. sowie IV. bis VIII. Nebengruppe und denjenigen Ether-Derivaten von substituierten 1-Hydroxyalkyl-azolen der Formel (I), in denen die Substituenten B, $R^1$, $R^2$ und $Z_m$ die Bedeutungen haben, die bereits für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich won solchen Säuren ableiten, die zu physiologisch verträglichen Additionsprodukten führen. Bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man beispielsweise das Natriumalkanolat des 2-(4-Chlorphenoxymethyl)-3,3-dimethyl-1-(1,2,4-triazol-1-yl)-butan-2-ols und Iodmethan als Ausgangsstoffe, so kann der Ablauf des erfindungsgemäßen Verfahrens durch das folgende Formelschema wiedergegeben werden:

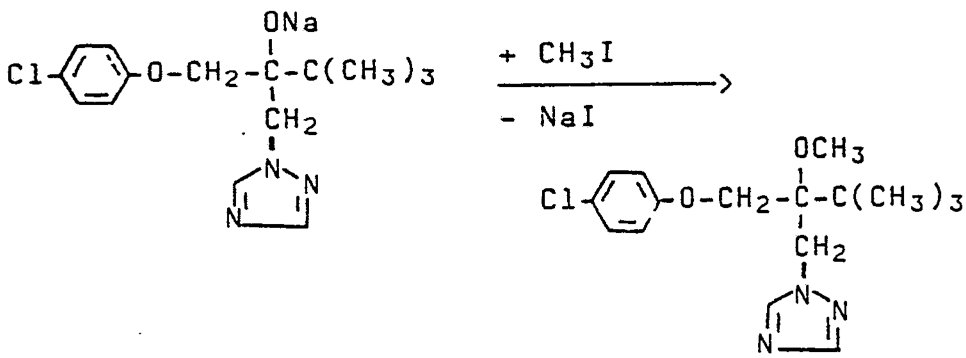


Die bei der Durchführung des erfindungsgemäßen Verfahrens als Ausgangsstoffe zu verwendenden Alkoholate von 1-Hydroxy-alkylazolen sind durch die Formel (II) allgemein definiert.

In dieser Formel stehen B, $R^1$, Z und der Index m für die Bedeutungen, die bereits im Zusammenhang mit der Beschreibung der erfindungsgemäßen Stoffe der Formel (I) für diese Substituenten bzw. für den Index m genannt wurden. M steht vorzugsweise für Alkalimetalle Lithium, Natrium und Kalium. M steht außerdem vorzugsweise für folgende quarternäre Ammoniumgruppen: Tetra-butylammonium, N-Benzyl-N,N,N-trimethylammonium, Hexadecyltrimethylammonium, Tetraethylammonium, Tetramethylammonium, Methyltrioctylammonium, N-Phenyl-N,N,N-trimethylammonium, N-(4-Methylbenzyl)-N,N,N-trimethylammonium, N-Benzyl-N,N-dimethyl-N-dodecylammonium, N,N-Dibenzyl-N,N-dimethylammonium, Benzyl-dimethyl-n-hexadecylammonium, Benzyl-tributylammonium, Benzyl-triethylammonium, Butyl-tripropylammonium, Octadecyltrimethylammonium, Tetrahexylammonium, Tetraoctylammonium und Hexadecylpyridinium; sowie vorzugsweise für folgende Phosphoniumgruppen: Tetraphenylphosphonium, Hexadecyltributylphosphonium, Ethyl-triphenylphosphonium oder Methyl-triphenylphosphonium.

Die Alkoholate der Formel (II) sind noch nicht bekannt. Sie können jedoch in allgemein bekannter Art und Weise erhalten werden, indem man die entsprechenden substituierten 1-Hydroxyalkyl-azole der Formel

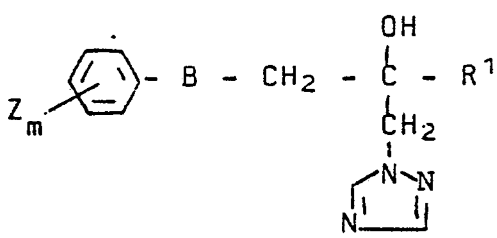 (IV)

in welcher

B,$R^1$,Z und m die oben angegebene Bedeutung haben,

mit geeigneten starken Basen, wie Alkalimetall-amiden oder -hydriden, quarternären Ammoniumhydroxiden oder Phosphoniumhydroxiden in einem indifferenten Lösungsmittel, wie beispielesweise Dioxan, bei Raumtemperatur umsetzt.

Die substituierten 1-Hydroxyalkyl-azole der Formel (IV) sind teilweise bekannt (vergleiche DE-OS 30 18 866, teilweise sind sie Gegenstand einer parallelen Patentanmeldung. Sie werden erhalten, indem man Oxirane der Formel

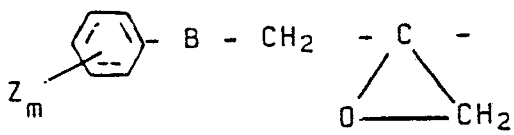 (V)

in welcher
B, $R^1$, Z und m die oben angegebene Bedeutung haben,
mit 1,2,4-Triazol der Formel

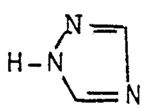 (VI)

in Gegenwart eines inerten organischen Lösungsmittels, wie beispielsweise Ethanol, und gegebenenfalls in Gegenwart einer Base, wie beispielsweise Natriumethylat, gegebenenfalls unter einem Druck von 1 bis 24 bar, bei Temperaturen zwischen 60 und 150°C umsetzt, oder Azolylmethyl-oxirane der Formel

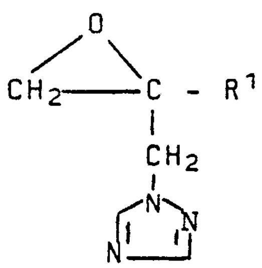 (VII)

in welcher
$R^1$ die oben angegebene Bedeutung hat,
mit (Thio)Phenolen der Formel

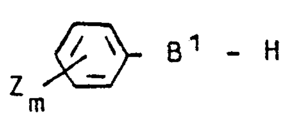 (VIII)

in welcher
Z und m die oben angegebene Bedeutung haben und
$B^1$ für Sauerstoff oder Schwefel steht,
in Gegenwart eines inerten organischen Lösungsmittels, wie beispielsweise Ethanol, und gegebenenfalls in

Gegenwart einer Base, wie beispielsweise Natriumethylat, gegebenenfalls unter einem Druck von 1 bis 25 bar, bei Temperaturen zwischen 60 und 100° C umsetzt.

Die Oxirane der Formel (V) sind teilweise bekannt (vergleiche DE-OS 30 18 866, teilweise sind sie Gegenstand einer parallen Patentanmeldung. Sie werden erhalten, indem man Ketone der Formel

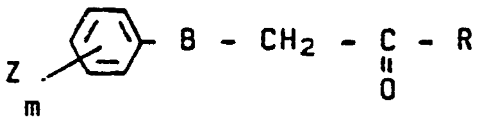 (IX)

in welcher
B,R,Z und m die oben angegebene Bedeutung haben,
entweder
α) mit Dimethyloxosulfonium-methylid der Formel

$$(CH_3)_2\overset{+}{S}O\overset{-}{C}H_2 \qquad (X)$$

in an sich bekannter Weise in Gegenwart eines Verdünnungsmittels, wie beispielsweise Dimethylsulfoxid, bei Temperaturen zwischen 20 und 80°C umsetzt (vergleiche hierzu auch die Angaben in J.Am.Chem.Soc. 87, 1363-1364 (1965)), oder
β) mit Trimethylsulfonium-methylsulfat der Formel

$$\left[(CH_3)_3S\right]^{(+)} \quad CH_3SO_4^{(-)} \qquad (XI)$$

in an sich bekannter Weise in Gegenwart eines inerten organischen Lösungsmittels, wie z.B. Acetonitril, und in Gegenwart einer Base, wie z.B. Natriummethylat, bei Temperaturen zwischen 0 bis 60°C, vorzugsweise bei Raumtemperatur, umsetzt (vergleiche auch die Angaben in Heterocycles 8, 397, (1977)).

Die so erhaltenen Oxirane der Formel (V) können gegebenenfalls ohne Isolierung direkt weiter umgesetzt werden.

Die bei der Herstellung der Oxirane der Formel (V) als Ausgangsstoffe benötigten Ketone der Formel (IX) sind bekannt (vergleiche z.B. DE-PS 22 01 063, DE-OS 26 32 603, DE-OS 26 32 602, DE-OS 26 35 664, DE-OS 26 35 666, DE-OS 27 05 678, DE-OS 29 18 894, DE-OS 29 18 893, DE-PS 22 01 063, DE-OS 27 05 678, DE-OS 27 37 489), bzw. sind sie Gegenstand eigener älterer Patentanmeldungen (vgl. die Deutschen Patentanmeldungen p 30 21 551 vom 7.6. 1980, P 31 19 390 vom 15.5.81, P 31 01 143 vom 16.1.1981), bzw. lassen sie sich nach im Prinzip bekannten Verfahren herstellen.

Das bei der Verfahrensvariante (α) benötigte Dimethyloxosulfoniummethylid der Formel (X) ist ebenfalls bekannt (vergleiche J.Am.Chem.Soc. 87, 1363-1364 (1965)). Es wird bei der obigen Umsetzung in frisch zubereitetem Zustand verarbeitet, indem man es in situ durch Umsetzung von Trimethylsulfoxosulfoniumiodid mit Natriumhydrid bzw. Natriumamid in Gegenwart eines Verdünnungsmittels erzeugt.

Das bei der Verfahrensvariante (β) benötigte Trimethylsulfonium-methylsulfat der Formel (XI) ist ebenfalls bekannt (vgl. Heterocycles 8, 397 (1977)). Es wird bei der obigen Umsetzung ebenfalls in frisch hergestelltem Zustand eingesetzt, indem man es durch Reaktion von Dimethylsulfid mit Dimethylsulfat in situ erzeugt.

Die auch als Ausgangsstoffe zur Herstellung der 1-Hydroxy-alkyl-azole der Formel (IV) zu verwendenden Azolylmethyloxirane der Formel (VII) sind noch nicht bekannt. Sie sind jedoch teilweise Gegenstand einer eigenen älteren Anmeldung (vgl. die Deutsche Patentanmeldung P 31 11 238 vom 21.3.1981), teilweise sind sie auch Gegenstand einer parallelen Patentanmeldung, bzw. können sie in allgemein bekannter Art und Weise erhalten werden, indem man Azolo-ketone der Formel

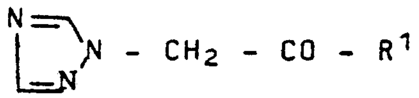 (XII)

in welcher
$R^1$ die oben angegebene Bedeutung hat
entsprechend den oben angegebenen Verfahrensvarianten (α) und (β) epoxidiert.

Die Azolo-ketone der Formel (XII) sind bekannt (vgl. DE-OS 24 31 407, DE-OS 26 38 470, DE-OS 28 20 361) bzw. sind sie Gegenstand einer eigenen älteren Patentanmeldung (vgl. die Deutsche Patentanmeldung P 30 48 266 vom 20.12.1980), bzw. lassen sie sich nach im Prinzip bekannten Verfahren herstellen.

Das außerdem als Ausgangsstoff zur Herstellung der l-Hydroxyalkyl-azole der Formel (IV) zu verwendende 1, 2, 4-Triazol der Formel (VI) bzw. die (Thio)Phenole der Formel (VII) sind allgemein bekannte Verbindungen der organischen Chemie.

Die weiterhin für das erfindungsgemäße Verfahren als Ausgangsstoffe zu verwendenden Halogenide sind durch die Formel (III) allgemein definiert. In dieser Formel steht $R^2$ für die Bedeutungen, die bereits im Zusammenhang mit der Beschreibung der erfindungsgemäßen Stoffe der Formel (I) für diesen Substituenten genannt wurden. Hal steht vorzugsweise für Chlor, Brom oder Iod.

Die Halogenide der Formel (III) sind allgemein bekannte Verbindungen der organischen Chemie.

Als Beispiele für die substituierten 1-Hydroxyalkyl-azole der Formel (IX), die den erfindungsgemäß als Ausgangsstoffe zu verwendenden Alkoholaten der Formel (II) zugrunde liegen, seien die folgenden Verbindungen der allgemeinen Formel

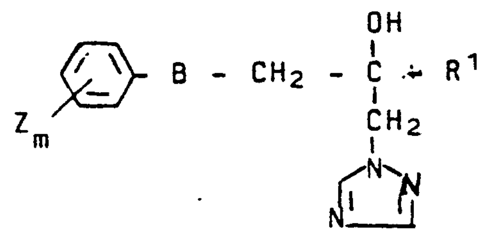 (IV)

genannt:

| $Z_m$ | B | $R^1$ |
|---|---|---|
| 4-Cl | O | $C(CH_3)_3$ |
| 4-Cl | $CH_2$ | $C(CH_3)_3$ |
| 4-Cl,2-$CH_3$ | O | $C(CH_3)_3$ |
| 2,4-$Cl_2$ | O | $C(CH_3)_3$ |
| 4-$CH_3$ | O | $C(CH_3)_3$ |
| 2-$CH_3$ | O | $C(CH_3)_3$ |
| 4-F | $CH_2$ | $C(CH_3)_3$ |
| 4-Cl | O | 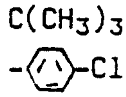 |
| 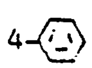 | O | $C(CH_3)_3$ |
| 2-Cl | O | $C(CH_3)_3$ |
| 2,4-$Cl_2$ | $CH_2$ | $C(CH_3)_3$ |

| $Z_m$ | B | $R^1$ |
|---|---|---|
| $2-CH_3$ | $CH_2$ | $C(CH_3)_3$ |
| 4-Cl | O | 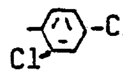-Cl |
| 3-Cl | O | $C(CH_3)_3$ |
| $3,4-Cl_2$ | O | $C(CH_3)_3$ |
| $4-CH_3$ | $CH_2$ | $C(CH_3)_3$ |
| 4-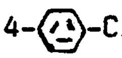-Cl | O | $C(CH_3)_3$ |
| - | O | $C(CH_3)_3$ |
| $4-OCH_3$ | O | $C(CH_3)_3$ |
| $4-C(CH_3)_3$ | O | $C(CH_3)_3$ |
| $4-OCF_3$ | O | $C(CH_3)_3$ |
| 4-Cl | O | $-C(CH_3)_2CH_2OCH_3$ |
| 4-Cl | S | $C(CH_3)_3$ |
| 4-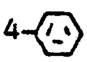 | O | $-C(CH_3)_2CH=CH_2$ |
| $4-CH_3$ | O | $-C(CH_3)_3CH_2F$ |
| 4-Cl | O | $-C(CH_3)_2CH_2F$ |
| $4-Cl,2-CH_3$ | O | $-C(CH_3)_2CH_2F$ |
| $2,4-Cl_2$ | O | $-C(CH_3)_2CH_2F$ |
| 4-Cl | S | $-C(CH_3)_2CH_2F$ |
| 2-Cl | S | $-C(CH_3)_2CH_2F$ |
| $3,4-Cl_2$ | S | $-C(CH_3)_2CH_2F$ |
| 4-Cl | O | $-C(CH_2F)_2CH_3$ |
| $2,4-Cl_2$ | O | $-C(CH_2F)_2CH_3$ |
| $4-Cl,2-CH_3$ | O | $-C(CH_2F)_2CH_3$ |
| $2,4-Cl_2$ | O | $-C(CH_3)_2CH_2OCH_3$ |
| $4-Cl, 2-CH_3$ | O | $-C(CH_3)_2CH_2OCH_3$ |
| 4-Cl | O | $-C(CH_3)_2CH_2OC_2H_5$ |
| 4-Cl | O | $-C(CH_3)_2CH_2O-$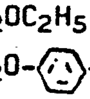-Cl |
| $2,4-Cl_2$ | O | $-C(CH_3)_2-$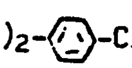-Cl |
| 4-Cl | O | $-C(CH_3)_2CH=CH_2$ |
| $2,4-Cl_2$ | O | $-C(CH_3)_2CH=CH_2$ |

| $Z_m$ | B | $R^1$ |
|---|---|---|
| 4-Cl | $CH_2$ | $-C(CH_3)_2CH_2F$ |
| 2,4-$Cl_2$ | $CH_2$ | $-C(CH_3)_2CH_2F$ |
| 2,4-$Cl_2$ | $CH_2$ | $-C(CH_2F)_2CH_3$ |
| 4-Cl | $CH_2$ | $-C(CH_2F)_2CH_3$ |
| 4-Cl | $CH_2$ | $-C(CH_3)_2CH_2O-$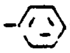-Cl |
| 4-Cl | $CH_2$ | $-C(CH_3)_2O-$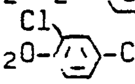-Cl |
| 3,4-$Cl_2$ | S | $-C(CH_3)_3$ |

Als Verdünnungsmittel kommen für die erfindungsgemäße Umsetzung inerte organische Lösungsmittel infrage. Hierzu gehören vorzugsweise Ether, wie Diethylether oder Dioxan; aromatische Kohlenwasserstoffe, wie Benzol; in einzelnen Fällen auch chlorierte Kohlenwasserstoffe, wie Chloroform, Methylenchlorid oder Tetrachlorkohlenstoff; sowie Hexamethylphosphorsäuretriamid.

Die Reaktionstemperaturen können bei der Durchführung des erfindungsgemäßen Verfahrens in einem größeren Bereich variiert werden. Im allgemeinen arbeitet man zwischen 0 und 120°C, vorzugsweise zwischen 20 und 100°C.

Bei der Durchführung des erfindungsgemäßen Verfahrens setzt man auf 1 Mol des Alkoholats der Formel (II) vorzugsweise 1 bis 2 Mol Halogenid der Formel (III) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform wird zweckmäßigerweise so verfahren, daß man von einem substituierten 1-Hydroxy-alkyl-azol-Derivat der Formel (IV) ausgeht, letzteres in einem geeigneten organischen Lösungsmittel mittels Alkalimetall-hydrid oder Alkalimetall-amid in das Alkalimetallalkoholat der Formel (II) überführt und letzteres ohne Isolierung sofort mit einem Halogenid der Formel (III) umsetzt, wobei unter Austritt von Alkalihalogenid die erfindungsgemäßen Verbindungen der Formel (I) in einem Arbeitsgang erhalten werden.

Nach einer weiteren bevorzugten Ausführungsform werden zweckmäßigerweise die Herstellung der Alkoholate der Formel (II) sowie die erfindungsgemäße Umsetzung in einem Zweiphasensystem, wie beispielsweise wässrige Natronoder Kalilauge/Toluol oder Methylenchlorid, unter Zusatz von 0,01-1 Mol eines Phasen-Transfer-Katalysators, wie beispielsweise Ammonium- oder Phosphoniumverbindungen, durchgeführt, wobei in der organischen Phase oder an der Grenzfläche die Alkoholate mit den in der organischen Phase befindlichen Halogeniden umgesetzt werden.

Zur Herstellung von physiologisch verträglichen Säureadditionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, 5chwefel-säure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure. Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I.bis II. sowie IV.bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindungen der Formel(I). Man kann Metallsalz-Komplexe in bekannter Weise, z.B. durch Abfiltrieren, isolieren und gegebenenfalls durch Umkristallisation reinigen.

Die erfindungsgemäß verwendbaren Wirkstoffe greifen in den Metabolismus der Pflanzen ein und können

deshalb als Wachstumsregulatoren eingesetzt werden.

Für die Wirkungsweise von Pflanzenwachstumsregulatoren gilt nach der bisherigen Erfahrung, daß ein Wirkatoff auch mehrere verschiedenartige Wirkungen auf Pflanzen susüben kann. Die Wirkungen der Stoffe hängen im wesentlichen ab von dem Zeitpunkt der Anwendung bezogen auf das Entwicklungsstadium dar Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung das Wuchses von krautigen und holzigen Pflanzen an Straßenrändern und in der. Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätaverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch iat es beispielsweise möglich, den Abbsu erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv teeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwunscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daG ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trckenheit oder hohen Salzgehalt des Bodens induziert werden. Hierdurch wird die Kultivierung von Pflanzen in Gebieten möglich, die hierzu normalerweise ungeeignet sind.

Die erfindungsgemäßen Wirkstoffe weisen eine starke mikrobizide Wirkung auf und können zur Bekämpfung von unervünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäß verwendeten Wirkstoffe mit besonders gutem Erfolg zur Bekämpfung solcher Pilze eingesetzt werden, die echte Mehltauerkrankungen hervorrufen; so zur Bekämpfung von Erysiphe-Arten, wie z.B. gegen den Erreger des Gerstenbzw. Getreidemehltaus (Erysiphe graminis), von Podosphaera-Arten, wie z.B. gegen den Erreger des Apfelmehltaus (Podosphaera leucotricha) und von Sphaerotheca-Arten, wie z.B. gegen den Erreger des Gurkenmehltaus (Sphaerotheca fuligenea), sowie auch zur Bekämpfung von Puccinia-Arten, wie z.B. gegen den Erreger des Braunrostes am Weizen (Puccinia recondita). Zu bemerken ist, daß die erfindungsgemäßen Stoffe auch eine breite fungizide in-vitro-Wirkung aufweisen.

In entsprechenden Aufwandmengen zeigen die erfindungs-gemäßen Wirkstoffe auch herbizide Eigenschaften.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sovie ULV-Formulierungen.

Diese Formulierungen werden in bekannter Weise hergestellt, z.B. durch vermischen der Wirkstoffe mit Streckmitteln, also flüssigen Lösungsmitteln, unter Druck stehenden verflüssigten Gasen und / oder festen Trägerstoffen, gegebenenfalls unter Verwendung von oberflächenaktiven Mitteln, also Emulgiermitteln und / oder Dispergiermitteln und / oder schaumerzeugenden Mitteln. Im Falle der Benutzung von Wasser als Streckmittel können z.B. auch organische Lösungsmittel als Hilfslösungsmittel vervendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark pclare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und / oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Garboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsveise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen Schäume, Suspensionen, Spritzpulver, Pasten, lösliche Pulver, Stäubemittel und Granulate, angevendet werden. Die Anwendung geschieht in üblicher Weise, z.B. durch Gießen, Verspritzen, Versprühen, Verstreuen, Verstäuben,

Verschäumen, Bestreihen usw. Es ist ferner möglich, die Wirkstoffe nach dem Ultra-Lowvolume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden.Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegatativen Gegebenheiten richtet.

Auch beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50g je Kilogramm Saatgut, vorzugsweise 0,01 bis 10g, benötigt. Bei Behandlung des Bodens sind Wirkstoffkonzentrationen von 0,00001 bis 0,1 Gew.-%, vorzugsweise von 0,0001 bis 0,02 % am Wirkungsort erforderlich.

**Herstellungsbeispiele**

**Beispiel I**

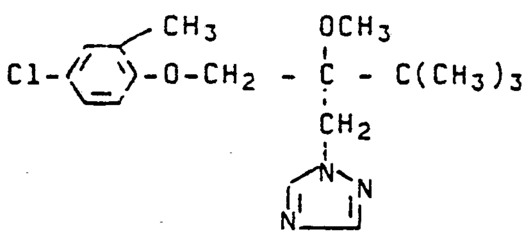

27g (0,9 Mol) 80 %-iges Natriumhydrid werden bei Raumtemperatur portionsweise in eine Lösung von 290g(0,86 Mol) 2-(4-Chlor-2-methyl-phenoxy-methyl)-3,3-dimethyl-1-(1,2,4-triazol-1-yl)-2-butanol in 1600 ml absolutem Dioxan eingetragen. Man läßt das Gemisch 4 Stunden bei Raumtemperatur nachrühren und tropft anschließend 141,9g (1 Mol) Iodmethan zu. Das Reaktionsgemisch wird 12 Stunden bei 40°C gerührt und danach erneut potionsweise mit 10g (0,33 Mol) 80%-igem Natriumhydrid versetzt. Nach dreistündigem Rühren bei Raumtemperatur werden 57 g (0,4 Mol) Iodmethan zugegeben und das Reaktionsgemisch 72 Stunden bei Raumtemperatur nachgerührt. Die Suspension wird filtriert, das Filtrat eingeengt, der ölige Rückstand in Dichlormethan aufgenommen, zweimal mit Wasser gewaschen, über Natriumsulfat getrocknet und eingeengt. Der Rückstand wird in Aceton mit 1,5-Naphthalindisulfonsäure versetzt, das ausfallende Salz wird abgesaugt, mit Aceton gewaschen, in Dichlormethan suspendiert und mit gesättigter Natriumhydrogencarbonatlösung versetzt. Die organische Phase wird abgetrennt, gewaschen und eingeengt. Man erhält 155g (54 % der Theorie) 2-(4-Chlor-2-methyl-phenoxymethyl)-3,3-dimethyl-2-methoxy-1-(1,2,4-triazol-1-yl)-butan als hellgelbes Oel vom Brechungsindex $n^{20}_D = 1,5390$.

In analoger Weise und entsprechend dem erfindungsgemäßen Verfahren werden die nachfolgenden Verbindungen der allgemeinen Formel

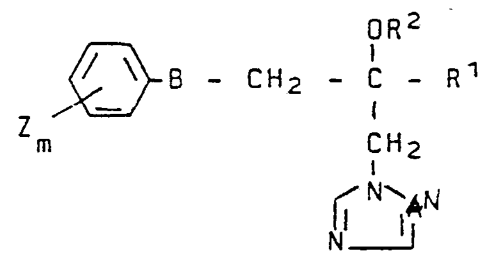

(I)

erhalten:

| Bsp. Nr.: | $Z_m$ | B | $R^1$ | $R^2$ | Fp(°C) bzw. $n_D^{20}$ |
|---|---|---|---|---|---|
| 2 | 4-Cl | O | $C(CH_3)_3$ | $CH_3$ | 198-203 (x HCl) |
| 3 | 2,4-$Cl_2$ | O | $C(CH_3)_3$ | $CH_3$ | 1,5382 |
| 4 | 4-Cl | $CH_2$ | $C(CH_3)_3$ | $CH_3$ | 1,5354 |
| 5 | 4-F | $CH_2$ | $C(CH_3)_3$ | $CH_3$ | 1,5212 |

**Verwendungsbeispiele**

In den nachfolgenden Verwendungsbeispielen werden die nachstehend angegebenen Substanzen als Vergleichsverbindungen eingesetzt:

(A) 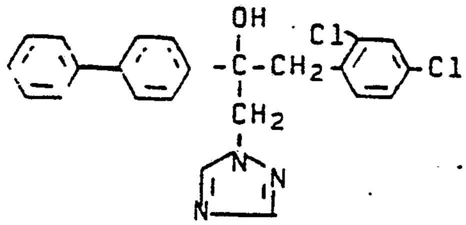

(B) 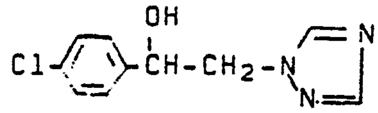

(C) 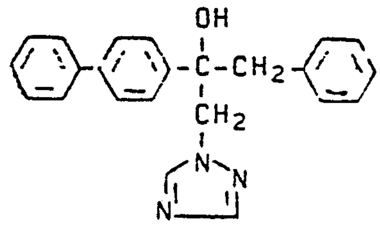

(D) 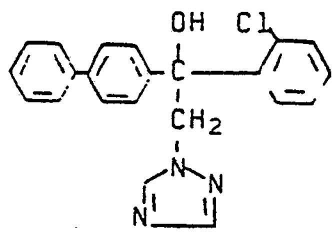

(E) 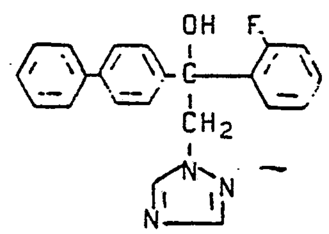

**Beispiel A**

Sphaerotheca-Test (Gurke) / protektiv
Lösungsmittel: 0,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca.75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1,3 und 4.

**Beispiel B**

Podosphaera-Test (Apfel) / protektiv
Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man jungePflanzen mit der Wirkstoffzubereitung bis zur Tropfnässe. Nach Antrocknen des Spritzbelages werden die Pflanzen durch Bestäuben mit Konidien des Apfelmehltauerregers (Podosphaera leucotricha) inokuliert.

Die Pflanzen werden dann im Gewächshaus bei 23°C und einer relativen Luftfeuchtigkeit von ca. 70 % aufgestellt.

9 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1,2,3 und 4.

**Beispiel C**

Erysiphe-Test (Gerste) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20° C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 2,1,3 und 4.

**Beispiel D**

Puccinia-Test (Weizen) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 2,1,3 und 4.

**Beispiel E**

Erysiphe-Test (Gerste) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 x 12 Korn 2 cm tief in eine Standarderde. 7 Tage nach der Aussaat, wenn die jungen

Pflanzen ihr erstes Blatt entfaltet haben, werden sie mit Sporen von Erysiphe graminis f. sp. hordei bestäubt.

Die Pflanzen werden in einem Gewächshaus bei einer Temperatur von ca. 20° C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 2,1,3 und 4.

**Beispiel F**

**Wuchsbeeinflussung bei Zuckerrüben**

Lösungsmittel: 30 Gewichtsteile Dimethylformamid
Emulgator: 1 Gewichtsteile Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit der Wirkstoffzubereitung besprüht. Nach 14 Tagen wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend dem der Kontrollpflanzen. Negative Werte kennzeichnen eine Wuchshemmung, positive eine Wuchsförderung gegenüber den Kontrollpflanzen.

Die erfindungsgemäßen Wirkstoffe 2, 1, 3 und 4 zeigen in diesem Test eine stärkere Wuchsbeeinflussung als die aus dem Stand der Technik bekannten Verbindungen (B), (D) und (E).

**Beispiel G**

**Wuchshemmung bei Baumwolle**

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf. Baumwollpflanzen werden im Gewächshaus bis zur vollen Entfaltung des 5.Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrolle berechnet. Es bedeuten 100 % Wuchshemmung den Stillstand des Wachstums und 0 % ein Wachstum entsprechend dem der Kontrollpflanzen.

Die erfindungsgemäßen Wirkstoffe 2, 3 und 1 zeigen in diesem Test eine bessere Wuchshemmung als die aus dem Stand der Technik bekannten Verbindungen (B), (D) und (E).

**Beispiel H**

**Wuchshemmung bei Sojabohnen**

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Die erfindungsgemäßen Wirkstoffe 2, 1, 3 und 4 zeigen in diesem Test eine stärkere Wuchshemmung als die aus dem Stand der Technik bekannten Verbindungen (A), (B) und (D).

**Patentansprüche**

1. Ether-Derivate von substituierten l-Hydroxy-alkylazolen der allgemeinen Formel

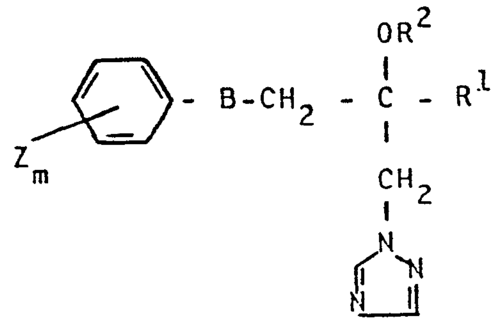

(I)

in welcher
$R^1$ für tert.-Butyl, Isopropyl oder Methyl steht, weiterhin für die Gruppierungen

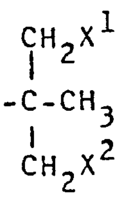

und

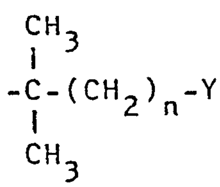

steht, sowie für gegebenenfalls durch Fluor, Chlor, Methyl oder Trifluormethyl ein- bis dreifach substituiertes Phenyl steht; wobei
$X^1$ für Wasserstoff, Fluor, Chlor oder Bron steht;
$X^2$ für Fluor, Chlor oder Brom steht;
Y für Methyl, Ethyl, Propyl, Methoxy, Ethoxy, Vinyl, sowie für gegebenenfalls eim- bis dreifach durch Chlor an den Phenyl-Resten substituiertes Phenyl oder Phenoxy steht;
n für die Zahlen 0 oder 1 steht;
Z für Fluor, Chlor, Brom, Methyl, tert.-Butyl, Methoxy Trifluornethyl, Trifluormethoxy, sowie für gegebenenfalls durch Chlor ein- bis dreifach substituiertes Phenyl steht;
$R^2$ für Methyl, Ethyl, n-Propyl, Isopropyl, n-Butyl Isobutyl, Allyl und Propargyl steht;
B für Schwefel oder die $CH_2$-Gruppe steht und weiterhin noch für Sauerstoff steht, sofern Z nicht Fluor bedeutet; und
m für die Zahlen 0, 1, 2 oder 3 steht;
sowie deren Säureadditions-Salze und Metallsalz-Komplexe.

2. Verfahren zur Herstellung von Ether-Derivaten von substituierten 1-Hydroxy-alkyl-azolen der allgemeinen Formel

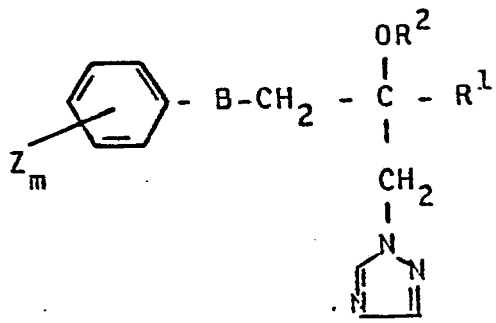

(I)

in welcher
$R^1$ für tert.-Butyl, Isopropyl oder Methyl steht,weiterhin für die Gruppierungen

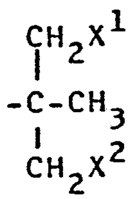

und

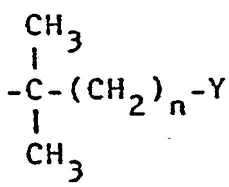

steht, sowie für gegebenenfalls durch Fluor, Chlor, Methyl oder Trifluormethyl ein- bis dreifach substituiertes Phenyl steht; wobei

$X^1$ für Wasserstoff, Fluor, Chlor oder Brom steht;

$X^2$ für Fluor, Chlor oder Brom steht;

Y für Methyl, Ethyl, Propyl, Methoxy, Ethoxy, Vinyl, sowie für gegebenenfalls ein- bi.s dreifach durch Chlor an den Phenyl-Resten substituiertes Phenyl oder Phenoxy steht;

n für die Zahlen 0 oder 1 steht;

Z für Fluor, Chlor, Brom, Methyl, tert.-Butyl, Methoxy Trifluormethyl, Trifluormethoxy, sowie für gegebenenfalls durch Chlor ein- bis dreifach substituiertes Phenyl steht;

$R^2$ für Methyl, Ethyl, n-Propyl, Isopropyl, n-Butyl Isobutyl, Allyl und Propargyl steht;

B für Schwefel oder die $CH_2$-Gruppe steht und weiterhin noch für Sauerstoff steht, sofern Z nicht Fluor bedeutet; und

m für die Zahlen 0, 1, 2 oder 3 steht;

dadurch gekennzeichnet, daß man Alkoholate von 1-Hydroxyalkyl-azolen der Formel

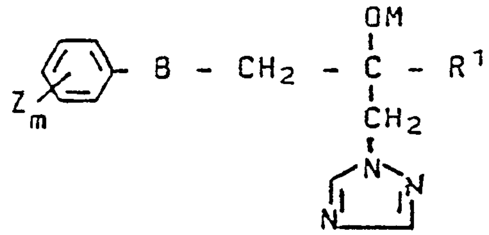

(II)

in welcher
B, $R^1$, Z und m die oben angegebene Bedeutung haben und
M für ein Alkalimetall, eine quarternäre Ammonium- oder Phosphoniumgruppe steht,
mit einem Halogenid der Formel
$R^2$ - Hal (III)
in welcher
$R^2$ die oben angegebene Bedeutung hat und
Hal für Halogen steht,
in Gegenwart eines Verdünnungsmittels umsetzt, und noch gegebenenfalls anschließend an die so erhaltenen Verbindungen der Formel (I) eine Säure oder ein Metallsalz addiert.

3. Pflanzenschutzmittel, gekennzeichnet durch einen Gehalt an mindestens einem Ether-Derivat von einem substituierten 1-Hydroxy-alkyl-azol der Formel (I) in Ansprüchen 1 und 2.

4. Fungizide und das Wachstum von Pflanzen regulierende Mittel, gekennzeichnet durch einen Gehalt an mindestens einem Ether-Derivat von einem substituierten 1-Hydroxy-alkyl-azol der Formel (I) in Ansprüchen 1 und 2.

5. Verwendung von Ether-Derivaten von substituierten 1-Hydroxy-alkyl-azloen der Formel (I) in Ansprüchen 1 und 2 zur Bekämpfung von Pilzen und zur Regulierung des Pflanzenwachstums.

**Claims**

1. Ether derivatives of substituted 1-hydroxyalkyl-azoles of the general formula

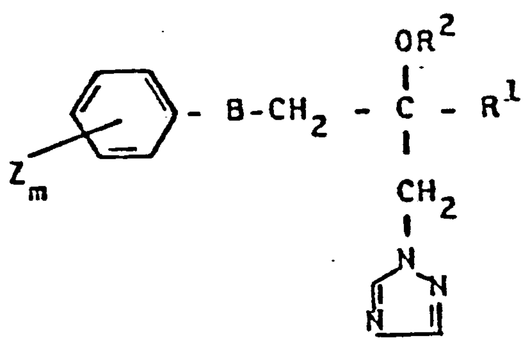

(I)

in which
$R^1$ represents tert.-butyl, isopropyl or methyl, also represents the groupings

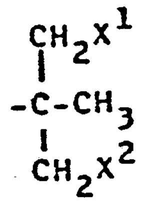 and 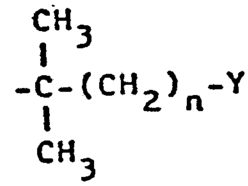

and represents phenyl which is optionally mono- to trisubstituted by fluorine, chlorine, methyl or trifluoromethyl; wherein

$X^1$ represents hydrogen, fluorine, chlorine or bromine;

$X^2$ represents fluorine, chlorine or bromine;

Y represents methyl, ethyl, propyl, methoxy, ethoxy, vinyl, and phenoxy or phenyl optionally mono- to trisubstituted on the phenyl radicals by chlorine;

n represents the numbers 0 or 1;

Z represents fluorine, chlorine, bromine, methyl, tert.-butyl, methoxy, trifluoromethyl, trifluoromethoxy and phenyl optionally mono- to trisubstituted by chlorine;

$R^2$ represents methyl, ethyl, n-propyl, isopropyl, n-butyl, isobutyl, allyl and propargyl;

B represents sulphur or the $CH_2$ group and also represents oxygen unless Z denotes fluorine; and

m represents the numbers 0, 1, 2 or 3;

and acid addition salts and metal salt complexes thereof.

2. Process for the preparation of ether derivatives of substituted 1-hydroxyalkyl-azoles of the general formula

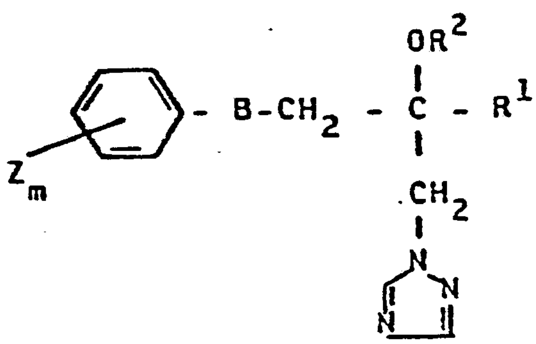

(I)

in which

$R^1$ represents tert.-butyl, isopropyl or methyl, also represents the groupings

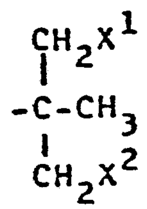 and 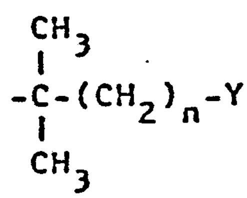

and represents phenyl which is optionally mono- to trisubstituted by fluorine, chlorine, methyl or trifluoromethyl; wherein

$X^1$ represents hydrogen, fluorine, chlorine or bromine;

$X^2$ represents fluorine, chlorine or bromine;

Y represents methyl, ethyl, propyl, methoxy, ethoxy, vinyl, and phenoxy or phenyl optionally mono- to trisubstituted on the phenyl radicals by chlorine;

n represents the numbers 0 or 1;

Z represents fluorine, chlorine, bromine, methyl, tert.-butyl, methoxy, trifluoromethyl, trifluoromethoxy, and phenyl optionally mono- to trisubstituted by chlorine;
$R^2$ represents methyl, ethyl, n-propyl, isopropyl, n-butyl, isobutyl, allyl and propargyl;
B represents sulphur or the $CH_2$ group and also represents oxygen unless Z denotes fluorine; and
m represents the numbers 0, 1, 2 or 3;
characterised-in that alcoholates of 1-hydroxyalkyl-azoles of the formula

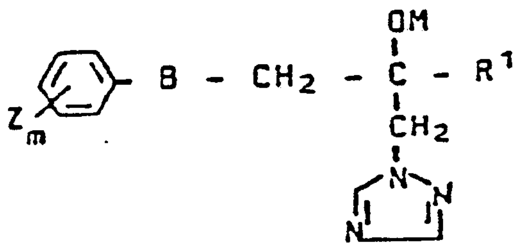

(II)

in which
B, $R^1$, Z and m have the abovementioned meaning and
M represents an alkali metal or a quaternary ammonium or phosphonium group,
are reacted with a halide of the formula
$R^2$ - Hal (III)
in which
$R^2$ has the abovementioned meaning and
Hal represents halogen,
in the presence of a diluent, and, if desired, an acid or a metal salt is also then added on to the compounds of the formula (I) thus obtained.

3. Plant-protecting agents, characterised in that they contain at least one ether derivative of a substituted 1-hydroxyalkyl-azole of the formula (I) in Claims 1 and 2.

4. Fungicides änd agents regulating the growth of plants, characterised in that they contain at least one ether derivative of a substituted 1-hydroxyalkyl-azole of the formula (I) in Claims 1 and 2.

5. Use of ether derivatives of substituted 1-hydroxyalkyl-azoles of the formula (I) in Claims 1 and 2 for combating fungi and for regulating plant growth.

**Revendications**

1. Dérivés, de type éther, de 1-hydroxy-alkylazoles substitués de formule générale

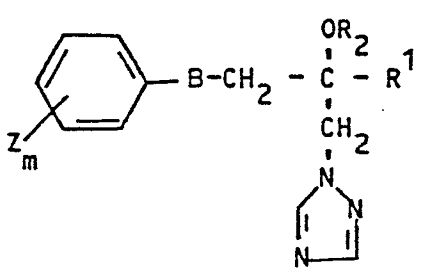

(I)

dans laquelle
$R^1$ représente un groupe tert-butyle, isopropyle ou méthyle, ou encore les groupements

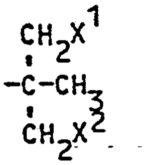

et

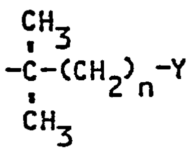

ainsi qu'un

groupe phényle éventuellement mono- à trisubstitué par le fluor, le chlore, les groupes méthyle ou trifluorométhyle;

$X^1$ représente l'hydrogène, le fluor, le chlore ou le brome;

$X^2$ représente le fluor, le chlore ou le brome;

Y représente un groupe méthyle, éthyle, propyle, méthoxy, éthoxy, vinyle, ou bien phényle ou phénoxy éventuellement mono- à trisubstitués par le chlore sur les noyaux phényle;

n est égal à 0 ou 1;

Z représente le fluor, le chlore, le brome, un groupe méthyle, tert-butyle, méthoxy, trifluorométhyle, trifluorométhoxy ou phényle éventuellement mono- à trisubstitué par le chlore;

$R^2$ représente un groupe méthyle, éthyle, n-propyle, isopropyle, nbutyle, isobtutyle, allyle et propargyle;

B represente le souf re ou le groupe $CH_2$, ou bien encore l'oxygène, lorsque Z ne représente pas le fluor; et

m est égal à 0, 1, 2 ou 3;

ainsi que leurs sels formés par addition avec des acides et complexes de sels métalliques.

2. Procédé de préparation de dérivés, de type éther, de 1-hydroxy-alkylazoles substitués de formule générale

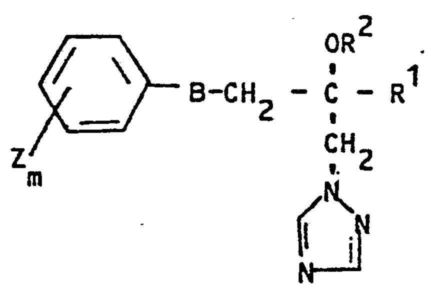

(I)

dans laquelle

$R^1$ représente un groupe tert-butyle, isopropyle ou méthyle, ou bien encore les groupements

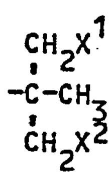

et

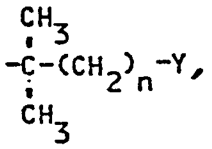

ou bien un

groupe phényle éventuellemnt mono- à trisubstitué par le fluor, le chlore, un groupe méthyle ou trif luorométhyle;

$X^1$ représente l'hydrogène, le fuor, le chlore ou le brome;

$X^2$ représente le fluor, le chlore ou le brome,

Y représente un groupe méthyle, éthyle, propyle, méthoxy, éthoxy, vinyle ou bi en encore phényle ou phénoxy éventuellement monoà trisubstitue par le chlore sur les noyaux phényles;

n est égal à 0 ou 1;

Z représente le fluor, le chlore, le brome, un groupe méthyle, tert-butyle, méthoxy, tri f luorométhyle, tri f luorométhoxy, ou bien phényle éventuellement mono- à trisubstitué par le chlore;

$R^2$ represente un groupe methyle, éthyle, n-propyle, isopropyle, n-butyle, isobutyle, allyle et propargyle;

B represente le soufre ou le groupe $CH_2$ ou bien encore l'oxygène à condition que Z ne représente pas le fluor; et

m est egal à 0, 1, 2 ou 3,

caractérisé en ce que l'on fait réagir des alcoolates de 1-hydroxy-alkylaz o les de formule

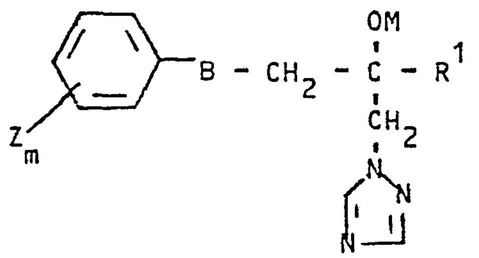

(II)

dans laquelle

B, $R^1$, Z et m ont les significations indiquées ci-dessus et

M représente un métal alcalin, un groupe ammonium ou phosphonium quaternaire,

avec un halogénure de formule

$R^2$ - Hal (III)

dans laquelle $R^2$ à les significations indiquées ci-dessus et Hal représente un halogene,

en présence d'un diluant, aprés quoi, le cas échéant, sur les composés de formule I ainsi obtenus, on fixé un acide ou un sel métallique par addition.

3. Produit phytosanitaire, caractérisé en ce qu'il contient au moins un dérivé, de type éther, d'un 1-hydroxy-alkylazole substitué de formule I des revendications 1 et 2.

4. Agent fongicide et régulateur de la croissance des vegétaux, caractérisé en ce qu'il contient au moins un dérive, de type éther, d'un 1-hydroxy-alkylazole substitué de formule I des revendications 1 et 2.

5. Utilisation des dérivés, de type éther, de 1-hydroxy-alkylazoles substitués de formule I des revendications 1 et 2 pour combattre les mycetes et pour la régulation de la croissance des végétaux.